# EUROPEAN PATENT APPLICATION

(11) **EP 4 834 223 A1**
(43) Date of publication of application: **04.11.2026**
(21) Application number: 23962991.8
(22) Date of filing: 25.12.2023
(51) Int. Cl.: A61B 5/1473, A61B 5/1486

(54) **BLOOD GLUCOSE LEVEL MEASUREMENT DEVICE**

(71) Applicant: NGK Corporation, Nagoya-shi, Aichi 467-8530 (JP)
(72) Inventor: FURUKAWA Masahiro, Nagoya-shi, Aichi 467-8530 (JP)
(74) Representative: Mewburn Ellis LLP
(86) International application number: PCT/JP2023/046315
(87) International publication number: WO 2025/141628

(57) **Abstract**

A blood glucose level measurement device includes a sensor portion that measures a blood glucose level of a user, a communication portion that transmits data representing the blood glucose level measured by the sensor portion to the outside, a flexible, sheet-shaped battery that supplies power to the sensor portion and the communication portion, a flexible, plate-shaped circuit portion that controls the operation of the communication portion, and an attachment portion configured to be attached to the body of the user. The battery has a peak current of 20 mA or more.

## Description

### Technical Field

The present disclosure relates to a blood glucose level measurement device.

### Background Art

Techniques related to devices for measuring blood glucose levels have been disclosed (see, for example, Patent Literature 1, Patent Literature 2, and Patent Literature 3).

### Citation List

### Patent Literature

Patent Literature 1: Japanese Translation of PCT International Application Publication No. 2021-522900
Patent Literature 2: Japanese Translation of PCT International Application Publication No. 2010-538745
Patent Literature 3: Japanese Translation of PCT International Application Publication No. 2023-541445

### Summary of Invention

### Technical Problem

Blood glucose level measurement devices worn on a body of a user for measuring blood glucose levels are used while being attached to the user's body, specifically to the skin of the user's arm or abdomen, for example. As the device is to be worn on the user's body for several days or several tens of days, it is desirable that the device provides a high level of fitting comfort against the user's skin. Furthermore, there is a demand for improved convenience in the processing of measured blood glucose level data and other tasks.

Therefore, one of the objects is to provide a blood glucose level measurement device that can improve convenience while enhancing the fitting comfort against the user's skin.

### Solution to Problem

A blood glucose level measurement device according to the present disclosure includes: a sensor portion that measures a blood glucose level of a user; a communication portion that transmits data representing the blood glucose level measured by the sensor portion to the outside; a flexible, sheet-shaped battery that supplies power to the sensor portion and the communication portion; a flexible, plate-shaped circuit portion that controls the operation of the communication portion; and an attachment portion configured to be attached to the body of the user. The battery has a peak current of 20 mA or more.

### Advantageous Effects of Invention

The blood glucose level measurement device as described above can improve convenience while enhancing the fitting comfort against the user's skin.

### Brief Description of Drawings

[FIG. 1] FIG. 1 is a schematic plan view of a blood glucose level measurement device in Embodiment 1.
[FIG. 2] FIG. 2 is a schematic side view of the blood glucose level measurement device shown in FIG. 1.
[FIG. 3] FIG. 3 is a block diagram showing a schematic configuration of the circuit portion included in the blood glucose level measurement device shown in FIG. 1.
[FIG. 4] FIG. 4 is a schematic diagram illustrating a state where the blood glucose level measurement device shown in FIG. 1 is attached to the skin of a user's arm.
[FIG. 5] FIG. 5 is a schematic plan view of a blood glucose level measurement device in Embodiment 2 of the present disclosure.
[FIG. 6] FIG. 6 is a schematic side view of the blood glucose level measurement device shown in FIG. 5.
[FIG. 7] FIG. 7 is a schematic plan view of a blood glucose level measurement device in Embodiment 3 of the present disclosure.
[FIG. 8] FIG. 8 is a schematic side view of the blood glucose level measurement device shown in FIG. 7.

### Description of Embodiments

### [Outline of Embodiments]

Embodiments of the present disclosure will be listed and described first. A blood glucose level measurement device according to the present disclosure includes: a sensor portion that measures a blood glucose level of a user; a communication portion that transmits data representing the blood glucose level measured by the sensor portion to the outside; a flexible, sheet-shaped battery that supplies power to the sensor portion and the communication portion; a flexible, plate-shaped circuit portion that controls the operation of the communication portion; and an attachment portion configured to be attached to the body of the user. The battery has a peak current of 20 mA or more.

According to the blood glucose level measurement device of the present disclosure, which includes the flexible, plate-shaped circuit portion and the flexible, sheet-shaped battery, the device can be appropriately fitted to the user's body when worn thereon. In other words, it provides a high level of fitting comfort against the user's skin. Furthermore, since the battery has a peak current of 20 mA or more, a high current can be supplied to the communication portion. Thus, with regard to the processing of blood glucose level data measured by the sensor portion, there is no requirement for the user to carry a data processing terminal with him/her; instead, the data can be transmitted to a data processing terminal located at a distant position. This eliminates the need for the user to carry the data processing terminal, which reduces the burden on the user for the processing of measured blood glucose level data, leading to improved convenience. As described above, such a blood glucose level measurement device can improve convenience while enhancing the fitting comfort against the user's skin.

In the blood glucose level measurement device of the above-described aspect, the communication portion may be arranged to overlap the battery when viewed in a thickness direction of the battery. With this configuration, even in cases where the communication portion becomes large due to the adoption of a plurality of communication antennas or other factors, the communication portion is arranged in an overlapping manner in an empty space located above the battery. This can reduce the area occupied by the blood glucose level measurement device as viewed in the thickness direction, thereby facilitating size reduction. Accordingly, it becomes easier to reduce the discomfort of the user when wearing the device and also reduce the risk of detachment of the device from the user's skin.

In the blood glucose level measurement device of the above-described aspect, the communication portion may be arranged adjacent to the battery when viewed in the thickness direction of the battery. This configuration facilitates reducing the thickness of the blood glucose level measurement device itself, thereby suppressing its size in the thickness direction. Accordingly, it becomes easier to reduce the discomfort of the user when wearing the device and also reduce the risk of detachment of the device from the user's skin. Furthermore, since the interference between the communication portion and the battery can readily be suppressed, it becomes easier to efficiently transmit radio waves over long distances. This can further improve the convenience.

In the blood glucose level measurement device of any of the above aspects, the circuit portion may be flat plate-shaped. The battery may be arranged between the circuit portion and the attachment portion. The communication portion may be arranged on the circuit portion. In such a blood glucose level measurement device where the communication portion and the battery are arranged on the front and back surfaces, respectively, of the circuit portion, the electronic components arranged on the surfaces of the circuit portion are low in density and relatively simplified in shape, which facilitates the production of the device and also the reduction of the total cost.

In the blood glucose level measurement device of any one of the above aspects, the peak current of the battery may be 30 mA or more. The communication portion may transmit the blood glucose level data using low power wide area (LPWA) communication. With this configuration, the blood glucose level data can be transmitted to a cloud server via LPWA, eliminating the need for a terminal on the receiving side. Furthermore, service providers that offer health management and the like using the blood glucose level measurement device take charge of the data processing and manipulations, making the user unnecessary to perform data upload and terminal manipulations. This can further improve the convenience.

In the blood glucose level measurement device of any one of the above aspects, the peak current of the battery may be 100 mA or more. The communication portion may transmit the blood glucose level data using at least one of LoRa (registered trademark) communication, Sigfox (registered trademark) communication, and Wi-Fi (registered trademark) communication. With this configuration, the blood glucose level data can be transmitted to a cloud server via at least one of the LoRa, Sigfox, and Wi-Fi communications, eliminating the need for a terminal on the receiving side. Furthermore, service providers that offer health management and the like using the blood glucose level measurement device take charge of the data processing and manipulations, making the user unnecessary to perform data upload and terminal manipulations. This can further improve the convenience.

In the blood glucose level measurement device of any one of the above aspects, the circuit portion may include a determination portion that determines whether data representing the blood glucose level measured by the sensor portion is within a predetermined range, and an alert issuance portion that issues an alert if the determination portion determines that the blood glucose level data is not within the predetermined range. With this configuration, the warning (alert) can notify the user wearing the blood glucose level measurement device of an abnormal blood glucose level. Accordingly, the user can readily be made aware of the abnormal blood glucose level.

In the blood glucose level measurement device of any one of the above aspects, the battery may have a capacity of 4 mAh or more. The blood glucose level measurement device may further include a display that displays data related to the blood glucose level. With this configuration, the display provides the user with a visual representation of the data regarding the blood glucose level. In this case, since the battery capacity is 4 mAh or more, it is possible to reduce the risk of insufficient battery capacity during the transmission of data by the communication portion, thereby promoting reliable data transmission.

In the blood glucose level measurement device of any one of the above aspects, the battery can be charged to at least 80% of the total capacity of the battery within 30 minutes. This makes it possible to reduce the period during which the absence of power hinders the measurement by the sensor portion and the data transmission by the communication portion.

In the blood glucose level measurement device of any one of the above aspects, the communication portion, the battery, and the circuit portion may be configured to be detachable from the blood glucose level measurement device. With this configuration, the user can recharge the battery by removing the portion including the battery, while leaving the sensor portion and the attachment portion on the user's skin when taking a shower, for example. This can further improve the convenience.

In the blood glucose level measurement device of any one of the above aspects, the battery may have a positive electrode and a negative electrode each composed of a sintered body. This configuration eliminates the use of combustible organic binders or conductive polymers as the materials constituting the electrodes, thereby improving the safety of the blood glucose level measurement device while achieving a smaller and thinner battery.

In the blood glucose level measurement device of any one of the above aspects, the blood glucose level measurement device may be sheet-shaped. The blood glucose level measurement device may have a rectangular outer shape with longitudinal and lateral lengths of 50 mm or less each when viewed in the thickness direction. The blood glucose level measurement device may have a thickness of 0.8 mm or more and 2.5 mm or less. The blood glucose level measurement device of this size can be worn on the user's body without causing any discomfort, allowing the user to continue daily activities with ease.

In the blood glucose level measurement device of any one of the above aspects, the sensor portion and the battery may be arranged adjacent to each other when viewed in a thickness direction of the blood glucose level measurement device. With this configuration, the sensor portion and the battery, both of which tend to increase in thickness, are positioned so as not to overlap each other, thereby allowing the device to be made thin as a whole. Accordingly, it becomes easier to reduce the discomfort of the user when wearing the device and also reduce the risk of detachment of the device from the user's skin.

### [Specific Embodiments]

Specific embodiments of the blood glucose level measurement device of the present disclosure will be described below with reference to the drawings. In the drawings referenced below, the same or corresponding portions are denoted by the same reference numerals and the description thereof will not be repeated.

### (Embodiment 1)

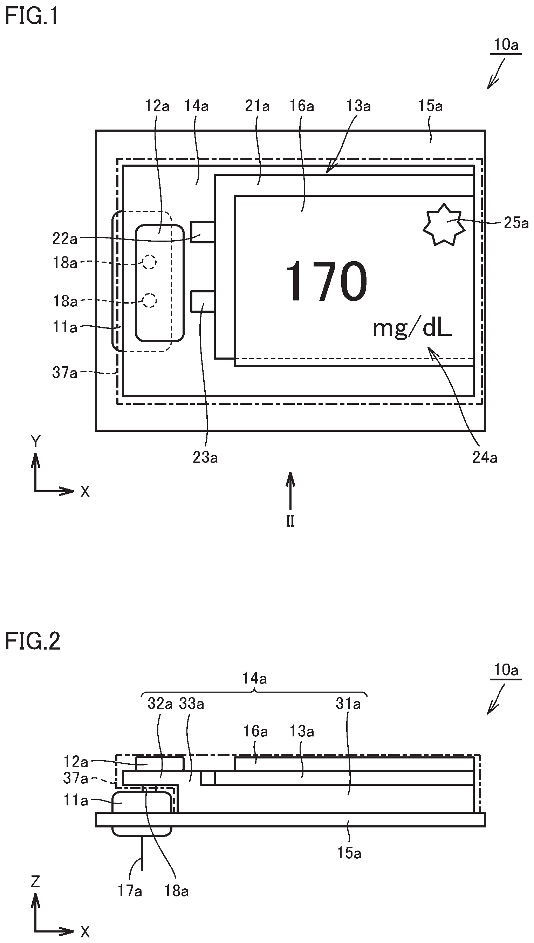

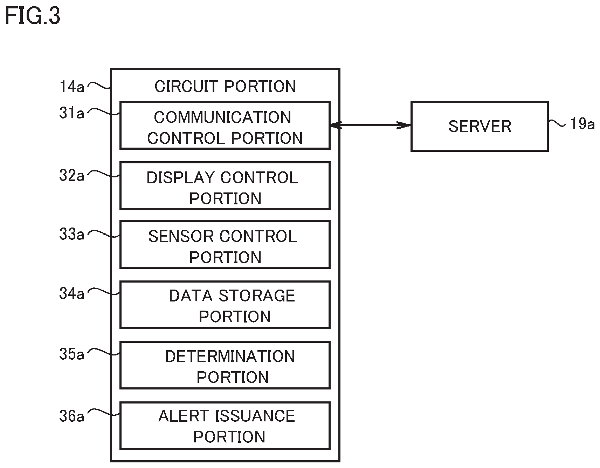

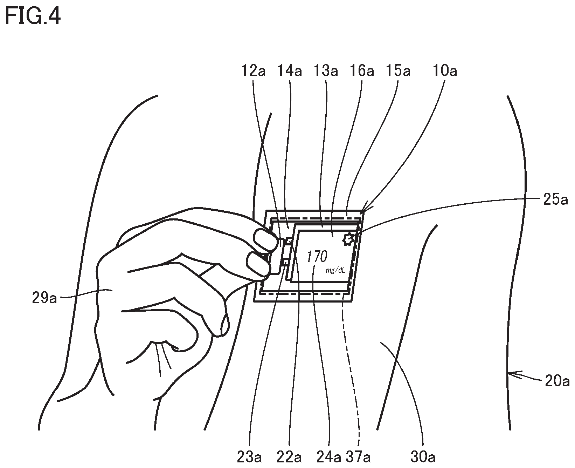

A blood glucose level measurement device according to the present disclosure will be described. FIG. 1 is a schematic plan view of a blood glucose level measurement device in Embodiment 1. FIG. 2 is a schematic side view of the blood glucose level measurement device shown in FIG. 1. FIG. 2 is a view of the blood glucose level measurement device in the direction indicated by arrow II in FIG. 1. FIG. 3 is a block diagram showing a schematic configuration of the circuit portion, which will be described later, included in the blood glucose level measurement device shown in FIG. 1. FIG. 4 is a schematic diagram illustrating a state where the blood glucose level measurement device shown in FIG. 1 is attached to the skin of a user's arm.

Referring to FIGS. 1, 2, 3, and 4, the blood glucose level measurement device 10a of Embodiment 1 is used while being directly adhered to the body of a user 20a, specifically to the skin of the user's arm, abdomen, or the like. FIG. 4 illustrates the state where the user 20a uses the hand 29a to apply the blood glucose level measurement device 10a to the arm 30a of the user 20a. The blood glucose level measurement device 10a remains attached to the skin of the user 20a for several days to several tens of days, during which the blood glucose level is measured periodically. The user 20a applies the blood glucose level measurement device 10a to a site on the body where the device will not obstruct normal daily activities. The blood glucose level measurement device 10a automatically measures the blood glucose levels periodically from the body of the user 20a.

In the present embodiment, the blood glucose level measurement device 10a is sheet-shaped. The blood glucose level measurement device 10a has a rectangular outer shape, when viewed in the thickness direction (Z direction), with four rounded corners. In the present embodiment, the direction along which the long side of the blood glucose level measurement device 10a extends is defined as the X direction, the direction along which the short side extends as the Y direction, and the thickness direction of the blood glucose level measurement device 10a is defined as the Z direction.

The blood glucose level measurement device 10a includes a sensor portion 11a, a communication portion 12a, a battery 13a, a circuit portion 14a, and an attachment portion 15a. In the present embodiment, the blood glucose level measurement device 10a also includes a display 16a.

The sensor portion 11a measures the user's blood glucose level. The sensor portion 11a includes a needle portion 17a that penetrates the skin of the user 20a. The needle portion 17a is sharp-pointed, and the blood glucose level measurement device 10a is invasive in the present embodiment. The sensor portion 11a uses the needle portion 17a to measure the blood glucose level from at least one of the user's blood and interstitial fluid.

The communication portion 12a has a rectangular outer shape, which is longer in the Y direction than in the X direction when viewed in the Z direction. Its four corners may be rounded. The communication portion 12a is also thin in thickness. The communication portion 12a transmits data representing the blood glucose level measured by the sensor portion 11a to the outside. The communication portion 12a is configured such that it can also receive external manipulation data (calibrations, measurement conditions, or changes to the display) using wireless communication. In other words, the communication portion 12a is capable of transmitting and receiving data to and from an external terminal via wireless communication. Specifically, the communication portion 12a can transmit data to and receive data from, for example, a server 19a that is located outside the blood glucose level measurement device 10a. For the communication portion 12a, a small-sized communication antenna, for example, is adopted. In the present embodiment, the communication portion 12a may transmit the blood glucose level data to the server 19a via a nearby gateway using low power wide area (LPWA) communication. Alternatively, the communication portion may transmit the data to both the server 19a and a portable receiver using both LPWA and Bluetooth low energy (BLE) communications. For the server 19a, a server in the cloud may be utilized.

The battery 13a is sheet-shaped and has flexibility. The battery 13a supplies power to the sensor portion 11a and the communication portion 12a. The battery 13a has a capacity of 4 mAh (milliampere-hours) or more. The battery 13a includes an exterior body 21a, a positive electrode tab terminal 22a, and a negative electrode tab terminal 23a. The exterior body 21a encloses a separator, an electrolyte, a positive electrode, a negative electrode (which are not shown), and others. In the present embodiment, the positive electrode tab terminal 22a and the negative electrode tab terminal 23a are spaced apart in the Y direction, each protruding from the exterior body 21a. The positive electrode tab terminal 22a and the negative electrode tab terminal 23a both protrude from the exterior body 21a in the direction opposite to the direction indicated by arrow Y. The battery 13a has at least the positive electrode composed of a sintered body. Such a battery 13a which does not use a combustible organic binder or conductive polymer as the material constituting the electrode can improve the safety of the blood glucose level measurement device while achieving a smaller and thinner battery.

In the present embodiment, the sensor portion 11a and the battery 13a are arranged adjacent to each other when viewed in the thickness direction (Z direction). Specifically, when viewed in the thickness direction, the sensor portion 11a and the battery 13a are arranged spaced apart in the X direction. In other words, the present embodiment provides a configuration in which there is no region where the sensor portion 11a and the battery 13a overlap with each other as viewed in the thickness direction.

The circuit portion 14a is plate-shaped and has flexibility. The circuit portion 14a has a thickness of 1.0 mm or less. In the present embodiment, the thickness of the circuit portion 14a is 0.2 mm or less. The circuit portion 14a is provided with an electric circuit such as a flexible printed circuit (FPC), which is not shown, and electrically connects the functional portions shown in FIG. 3 and controls the operations of the communication portion 12a. The circuit portion 14a includes a first region 26a, and a second region 27a that differs from the first region 26a in terms of its location in the thickness direction. A step 28a is provided between the first region 26a and the second region 27a.

The battery 13a and the display 16a are mounted on the first region 26a. The display 16a is arranged such that its display surface is exposed on the surface of the blood glucose level measurement device 10a. The area where the first region 26a is located is configured such that the members are stacked in the order of: the attachment portion 15a, the first region 26a of the circuit portion 14a, the battery 13a, and the display 16a.

The communication portion 12a is mounted on the second region 27a. In the area where the second region 27a is located, the sensor portion 11a is arranged between the attachment portion 15a and the circuit portion 14a. A portion of the sensor portion 11a where the needle portion 17a is provided is arranged on the lower side of the attachment portion 15a. The sensor portion 11a and the circuit portion 14a are connected by a connection terminal 18a. In the area where the second region 27a is located, the members are arranged in the order of: a portion of the sensor portion 11a including the needle portion 17a, the attachment portion 15a, the remaining portion of the sensor portion 11a, the connection terminal 18a, the second region 27a of the circuit portion 14a, and the communication portion 12a.

The circuit portion 14a includes a communication control portion 31a, a display control portion 32a, a sensor control portion 33a, a data storage portion 34a, a determination portion 35a, and an alert issuance portion 36a. The communication control portion 31a controls the operations of the communication portion 12a, including the timing of data transmission by the communication portion 12a. The display control portion 32a controls the display operations on the display 16a, including the generation of images to be displayed on the display 16a. The sensor control portion 33a controls the operations of the sensor portion 11a, including the measurement of the blood glucose level from at least one of the blood and interstitial fluid in contact with the needle portion 17a, in accordance with the timing of blood glucose level measurement. The data storage portion 34a, which is, for example, a memory provided in the circuit portion 14a, stores data on blood glucose levels and others. The determination portion 35a determines whether the blood glucose level data measured by the sensor portion 11a is within a predetermined range. The alert issuance portion 36a issues an alert if the determination portion 35a determines that the blood glucose level data is not within the predetermined range. The alert is issued, for example, via vibration, visible light, or sound, or by displaying a warning on the display 16a with text, images, or the like.

The attachment portion 15a is attached to the user's body. An example of the attachment portion 15a is an adhesive tape. The attachment portion 15a is positioned on the lowermost layer in the thickness direction, except for the region where a portion of the sensor portion 11a, including the needle portion 17a, is arranged.

The display 16a displays data related to the blood glucose levels. The display 16a is flat plate-shaped and has an electronic paper screen, for example. The display 16a is supplied with power from the battery 13a. The display 16a has a thickness of 1.0 mm or less. In the present embodiment, the thickness is 0.4 mm or less. As shown in FIG. 1, the display 16a is configured such that, when viewed in the thickness direction, its right-side end in the X direction is aligned with the ends in the X direction of the circuit portion 14a and the attachment portion 15a. It should be noted that in FIG. 1, the display 16a displays measurement data 24a of the blood glucose level reading: "170 mg/dL", and a warning symbol 25a indicating that the measured blood glucose level data has been determined to be outside the predetermined range by the determination portion 35a.

The blood glucose level measurement device 10a includes a covering portion 37a. The communication portion 12a, the battery 13a, the circuit portion 14a, the display 16a, and a portion of the connection terminal 18a are covered with the covering portion 37a. In FIG. 1 and other drawings, the covering portion 37a is shown with a long dashed short dashed line. The covering portion 37a is composed of a flexible resin. In other words, the communication portion 12a, the battery 13a, the circuit portion 14a, the display 16a, and a portion of the connection terminal 18a are coated with the covering portion 37a. The covering portion 37a is provided on the attachment portion 15a, except for the region where the sensor portion 11a is placed. The covering portion 37a is formed by lamination, molding, or the like. The display 16a has its display screen exposed from the covering portion 37a, or the visibility of the display screen of the display 16a is ensured by providing a transparent covering portion 37a. The members covered with the covering portion 37a are configured to be detachable from the attachment portion 15a. The provision of this covering portion 37a makes it possible to integrate the detachable portions into a single transmitter unit, for example, and also improves the waterproof, dustproof, and stainproof properties of the members covered.

The battery 13a is of a size where its longitudinal and lateral lengths in the footprint are 45 mm or less each, and its thickness is 1.5 mm or less. In the present embodiment, the footprint is 40 mm × 30 mm or less, and the thickness is 0.5 mm or less. The battery 13a has a peak current of 20 mA or more. In the present embodiment, the peak current of the battery 13a is 30 mA or more. The battery 13a can be charged to at least 80% of its total capacity within 30 minutes.

Conventional blood glucose level measurement devices may adopt small, non-rechargeable button batteries. Such small button batteries have a low peak current of, for example, 0.1 mA or less, which cannot support wireless communication alone. Thus, there was a need to additionally mount, for example, a capacitor to temporarily store power and release a high current. This would result in an increased thickness of the blood glucose level measurement device itself, due to at least one of the following two factors: the bulky capacitor, and the extra space required.

According to the blood glucose level measurement device 10a described above, which includes the flexible, plate-shaped circuit portion 14a and the flexible, sheet-shaped battery 13a, the device can be appropriately fitted to the user's body when worn thereon. In other words, it provides a high level of fitting comfort against the user's skin. Furthermore, since the battery 13a has a peak current of 20 mA or more, a high current can be supplied to the communication portion 12a. Thus, with regard to the processing of blood glucose level data measured by the sensor portion 11a, there is no requirement for the user to carry a data processing terminal with him/her; instead, the data can be transmitted to a data processing terminal located at a distant position. This eliminates the need for the user to carry the data processing terminal, which reduces the burden on the user for the processing of measured blood glucose level data, leading to improved convenience. As described above, this blood glucose level measurement device 10a can improve convenience while enhancing the fitting comfort against the user's skin.

In the present embodiment, the communication portion 12a is arranged adjacent to the battery 13a. Conventional blood glucose level measurement devices may adopt small, non-rechargeable button batteries. In such cases, although the footprint could be made smaller, the battery itself would have a thickness exceeding 2.5 mm, for example. When the above configuration where the communication portion 12a is arranged adjacent to the battery 13a is adopted, the blood glucose level measurement device 10a by itself can be made thinner, thereby suppressing its size in the thickness direction. Accordingly, it becomes easier to reduce the discomfort of the user when wearing the device and also reduce the risk of detachment of the device from the user's skin. Furthermore, since the interference between the communication portion 12a and the battery 13a can readily be suppressed, it becomes easier to efficiently transmit radio waves over long distances. This can further improve the convenience.

In the present embodiment, the peak current of the battery 13a is 30 mA or more. The communication portion 12a transmits the blood glucose level data using LPWA communication. Thus, the blood glucose level data can be transmitted to a server via LPWA, eliminating the need for a terminal on the receiving side. Furthermore, service providers that offer health management and others using the blood glucose level measurement device 10a take charge of the data processing and manipulations, making the user unnecessary to perform data upload and terminal manipulations. This can further improve the convenience.

In the present embodiment, the circuit portion 14a includes the determination portion 35a that determines whether data representing the blood glucose level measured by the sensor portion 11a is within a predetermined range, and the alert issuance portion 36a that issues an alert if the determination portion 35a determines that the blood glucose level data is not within the predetermined range. Therefore, the warning (alert) can notify the user wearing the blood glucose level measurement device 10a of an abnormal blood glucose level. Accordingly, the user can readily be made aware of the abnormal blood glucose level.

In the present embodiment, the battery 13a has a capacity of 4 mAh or more. The device further includes the display 16a that displays data related to the blood glucose level. Thus, the display 16a provides the user with a visual representation of the data regarding the blood glucose level. In this case, since the capacity of the battery 13a is 4 mAh or more, it is possible to reduce the risk of insufficient battery capacity during the transmission of data by the communication portion 12a, thereby promoting reliable data transmission.

In the present embodiment, the battery 13a can be charged to at least 80% of the total capacity of the battery 13a within 30 minutes. This makes it possible to reduce the period during which the absence of power hinders the measurement by the sensor portion 11a and the data transmission by the communication portion 12a.

In the present embodiment, the communication portion 12a, the battery 13a, and the circuit portion 14a are configured to be detachable from the blood glucose level measurement device 10a. This allows the user to recharge the battery by removing the portion including the battery 13a, while leaving the sensor portion 11a and the attachment portion 15a on the user's skin when taking a shower, for example. This can further improve the convenience.

In the present embodiment, the blood glucose level measurement device 10a is sheet-shaped. The blood glucose level measurement device 10a has a rectangular outer shape with longitudinal and lateral lengths of 50 mm or less each when viewed in the thickness direction. The blood glucose level measurement device 10a has a thickness of 0.8 mm or more and 2.5 mm or less. The blood glucose level measurement device 10a of this size can be worn on the user's body without causing any discomfort, allowing the user to continue daily activities with ease.

In the present embodiment, the sensor portion 11a and the battery 13a are arranged adjacent to each other when viewed in a thickness direction of the blood glucose level measurement device 10a. Thus, the sensor portion 11a and the battery 13a, both of which tend to increase in thickness, are configured so as not to overlap each other, thereby allowing the device to be made thin as a whole. Accordingly, it becomes easier to reduce the discomfort of the user when wearing the device and also reduce the risk of detachment of the device from the user's skin.

### (Embodiment 2)

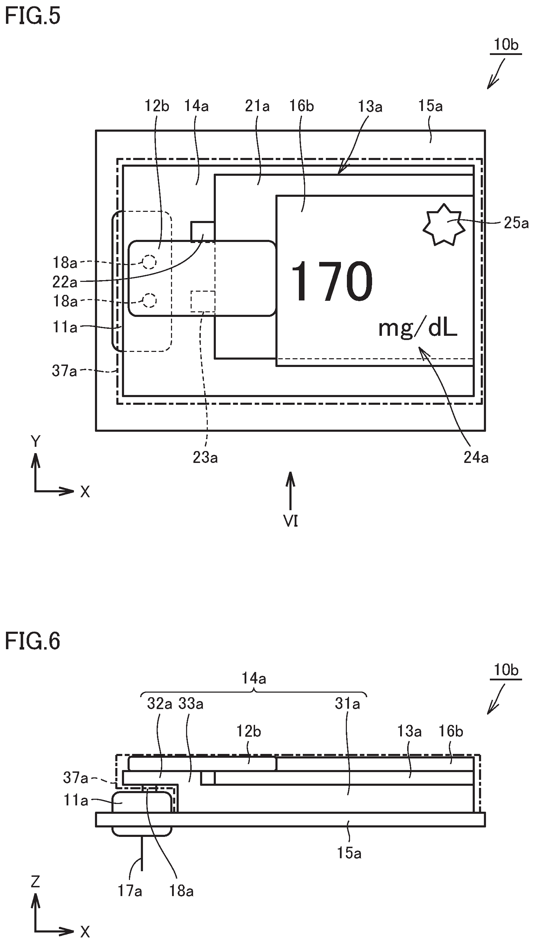

Another embodiment, Embodiment 2, will now be described. FIG. 5 is a schematic plan view of a blood glucose level measurement device in Embodiment 2 of the present disclosure. FIG. 6 is a schematic side view of the blood glucose level measurement device shown in FIG. 5. FIG. 6 is a view of the blood glucose level measurement device in the direction indicated by arrow VI in FIG. 5. The blood glucose level measurement device 10b of Embodiment 2 basically has a configuration similar to that of Embodiment 1, and exerts similar effects. However, the blood glucose level measurement device 10b of Embodiment 2 differs from that of Embodiment 1 in terms of the configuration of the communication portion and others.

Referring to FIGS. 5 and 6, the communication portion 12b included in the blood glucose level measurement device 10b of Embodiment 2 is arranged to overlap the battery 13a. In the present embodiment, the communication portion 12b has an outer shape identical to that of the communication portion 12a of Embodiment 1, and is arranged such that its length in the X direction is longer than the length in the Y direction. In other words, compared to the case of Embodiment 1, the communication portion 12b which is larger than the communication portion 12a is arranged. In the present embodiment, the communication portion 12b is arranged such that its part overlaps the battery 13a. It should be noted that a display 16b is configured to have a shorter length in the X direction than the display 16a of Embodiment 1. The configuration of the sensor portion 11a, the battery 13a, the circuit portion 14a, and the attachment portion 15a is similar to that of Embodiment 1.

With the configurations described above, even in cases where the communication portion 12b becomes large due to the adoption of a plurality of communication antennas (LPWA, BLE) or high-performance antennas, the communication portion 12b can be arranged in at least one of: the empty space located above the battery 13a, and the empty space created by reducing the size of the display 16a. This can avoid the increase in thickness of the blood glucose level measurement device 10b. In this case, even when the communication portion 12b, for example, becomes large, the dimension in the X direction of the blood glucose level measurement device 10b as a whole can be made identical to that shown in Embodiment 1. It should be noted that, assuming that the dimensions of the communication portion 12b remain unchanged, the dimension in the X direction of the blood glucose level measurement device 10b as a whole can be made smaller than that shown in Embodiment 1, which can reduce the user's discomfort and increase the number of possible attachment sites for the blood glucose level measurement device 10b.

### (Embodiment 3)

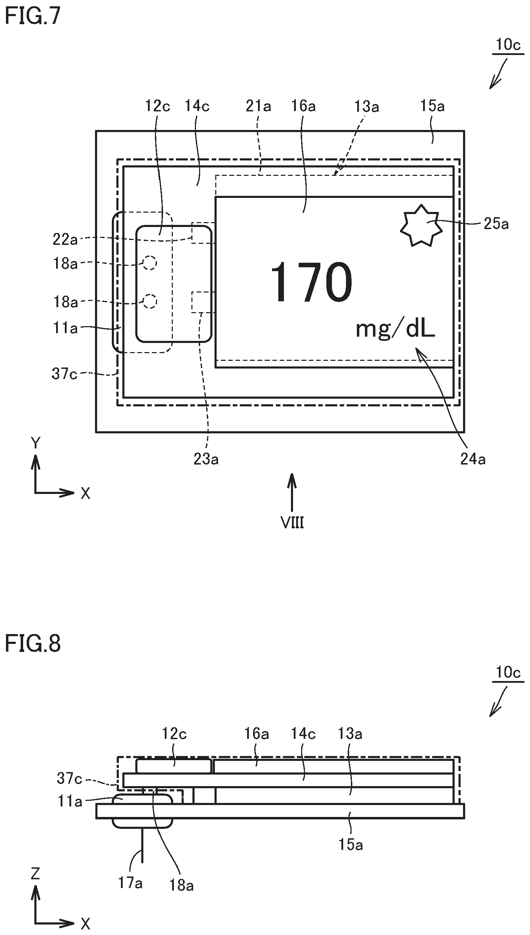

Yet another embodiment, Embodiment 3, will now be described. FIG. 7 is a schematic plan view of a blood glucose level measurement device in Embodiment 3 of the present disclosure. FIG. 8 is a schematic side view of the blood glucose level measurement device shown in FIG. 7. FIG. 8 is a view of the blood glucose level measurement device in the direction indicated by arrow VIII in FIG. 7. The blood glucose level measurement device 10c of Embodiment 3 basically has a configuration similar to that of Embodiment 1, and exerts similar effects. However, the blood glucose level measurement device 10c of Embodiment 3 differs from that of Embodiment 1 in terms of the configuration of the circuit portion and others.

Referring to FIGS. 7 and 8, the circuit portion 14c included in the blood glucose level measurement device 10c of Embodiment 3 is flat plate-shaped, which differs from those of Embodiments 1 and 2. In other words, the circuit portion 14c has a configuration that does not include any of the first region, second region, or step. In the blood glucose level measurement device 10c, unlike the cases of Embodiments 1 and 2, the attachment portion 15a, the battery 13a, the circuit portion 14a, and the display 16a are stacked in this order. That is, the battery 13a is arranged between the circuit portion 14a and the attachment portion 15a. A communication portion 12c is arranged on the circuit portion 14a. Here, the communication portion 12c has a shorter distance from the display 16a than the communication portion 12a of Embodiment 1, and has a shape with its longitudinal length slightly shorter than that of the communication portion 12a, communication portion 12b of Embodiment 2. In this case, the longitudinal length of a covering portion 37c also becomes shorter than in Embodiments 1 and 2. In this manner, the communication portion 12c of any shape can be arranged, depending on the number of communication antennas and communication capabilities required. When viewed in the thickness direction (Z direction) of the battery 13a, the communication portion 12c is arranged to overlap the battery 13a. Specifically, the communication portion 12c is arranged such that its part overlaps the battery 13a.

In such a blood glucose level measurement device 10c where the communication portion 12c and the battery 13a are arranged on the front and back surfaces, respectively, of the circuit portion 14a, the electronic components arranged on the surfaces of the circuit portion 14a are low in density and relatively simplified in shape, which makes it easier to produce the device and also facilitates reducing the total cost.

In the present embodiment, when viewed in the thickness direction of the battery 13a, the communication portion 12c is arranged to overlap the battery 13a. Therefore, even in cases where the communication portion 12c becomes large due to the adoption of a plurality of communication antennas or other factors, the communication portion 12c is arranged in an overlapping manner in an empty space located above the battery 13a. This can reduce the area occupied by the blood glucose level measurement device 10c as viewed in the thickness direction, thereby facilitating size reduction. In the present embodiment, the longitudinal length is shorter compared to the case of Embodiment 1. Accordingly, it becomes easier to reduce the discomfort of the user when wearing the device and also reduce the risk of detachment of the device from the user's skin.

### (Other Embodiments)

While the above embodiments have an electronic paper screen as a display, the present invention is not limited thereto; a liquid crystal screen or a light emitting diode (LED) lamp emitting visible light can be used for display. The alert issuance portion may issue an alert via vibration or sound. It is of course possible to issue an alert via vibration or sound in conjunction with display on the display.

Furthermore, in the above embodiments, the battery may have positive and negative electrodes both composed of a sintered body. This configuration eliminates the use of combustible organic binders or conductive polymers as the materials constituting the electrodes, thereby improving the safety of the blood glucose level measurement device while achieving a smaller and thinner battery.

In the above embodiments, the battery may have a peak current of 100 mA or more. The communication portion may transmit blood glucose level data using at least one of the LoRa communication, Sigfox communication, and Wi-Fi communication. In this manner, the blood glucose level data can be transmitted to a cloud server using at least one of the LoRa, Sigfox, and Wi-Fi communications, eliminating the need for a terminal on the receiving side. Furthermore, service providers that offer health management and the like using the blood glucose level measurement device take charge of the data processing and manipulations, making the user unnecessary to perform data upload and terminal manipulations. This can further improve the convenience.

While the above embodiments adopt, as the mechanism for measuring blood glucose levels, an (invasive) configuration where the sensor portion includes a needle portion which contacts at least one of the blood and interstitial fluid beneath the skin, the present invention is not limited thereto and may adopt a (non-invasive) configuration where the sensor portion does not include a needle portion and does not harm the skin.

It should be understood that the embodiments disclosed herein are illustrative and non-restrictive in every respect. The scope of the present invention is defined by the terms of the claims, rather than the description above, and is intended to include any modifications within the scope and meaning equivalent to the terms of the claims.

### Reference Signs List

10a, 10b, 10c: blood glucose level measurement device; 11a: sensor portion; 12a, 12b, 12c: communication portion; 13a: battery; 14a, 14c: circuit portion; 15a: attachment portion; 16a, 16b: display; 17a: needle portion; 18a: connection terminal; 19a: server; 20a: user; 21a: exterior body; 22a: positive electrode tab terminal; 23a: negative electrode tab terminal; 24a: measurement data; 25a: warning symbol; 26a: first region; 27a: second region; 28a: step; 29a: hand; 30a: arm; 31a: communication control portion; 32a: display control portion; 33a: sensor control portion; 34a: data storage portion; 35a: determination portion; 36a: alert issuance portion; and 37a, 37c: covering portion.

## Claims

1. A blood glucose level measurement device comprising:
a sensor portion that measures a blood glucose level of a user;
a communication portion that transmits data representing the blood glucose level measured by the sensor portion to the outside;
a flexible, sheet-shaped battery that supplies power to the sensor portion and the communication portion;
a flexible, plate-shaped circuit portion that controls the operation of the communication portion; and
an attachment portion configured to be attached to the body of the user;
the battery having a peak current of 20 mA or more.

2. The blood glucose level measurement device according to claim 1, wherein the communication portion is arranged to overlap the battery when viewed in a thickness direction of the battery.

3. The blood glucose level measurement device according to claim 1, wherein the communication portion is arranged adjacent to the battery when viewed in a thickness direction of the battery.

4. The blood glucose level measurement device according to any one of claims 1 to 3, wherein
the circuit portion is flat plate-shaped,
the battery is arranged between the circuit portion and the attachment portion, and
the communication portion is arranged on the circuit portion.

5. The blood glucose level measurement device according to any one of claims 1 to 4, wherein
the peak current of the battery is 30 mA or more, and
the communication portion transmits the blood glucose level data using LPWA communication.

6. The blood glucose level measurement device according to any one of claims 1 to 4, wherein
the peak current of the battery is 100 mA or more, and
the communication portion transmits the blood glucose level data using at least one of LoRa communication, Sigfox communication, and Wi-Fi communication.

7. The blood glucose level measurement device according to any one of claims 1 to 6, wherein the circuit portion includes
a determination portion that determines whether data representing the blood glucose level measured by the sensor portion is within a predetermined range, and
an alert issuance portion that issues an alert if the determination portion determines that the blood glucose level data is not within the predetermined range.

8. The blood glucose level measurement device according to any one of claims 1 to 7, wherein the battery has a capacity of 4 mAh or more,
the device further comprising a display that displays data related to the blood glucose level.

9. The blood glucose level measurement device according to any one of claims 1 to 8, wherein the battery is capable of being charged to at least 80% of the total capacity of the battery within 30 minutes.

10. The blood glucose level measurement device according to any one of claims 1 to 9, wherein the communication portion, the battery, and the circuit portion are configured to be detachable from the blood glucose level measurement device.

11. The blood glucose level measurement device according to any one of claims 1 to 10, wherein the battery has a positive electrode and a negative electrode each composed of a sintered body.

12. The blood glucose level measurement device according to any one of claims 1 to 11, wherein
the device is sheet-shaped,
the device has a rectangular outer shape with longitudinal and lateral lengths of 50 mm or less each when viewed in the thickness direction, and
the device has a thickness of 0.8 mm or more and 2.5 mm or less.

13. The blood glucose level measurement device according to any one of claims 1 to 12, wherein the sensor portion and the battery are arranged adjacent to each other when viewed in a thickness direction of the blood glucose level measurement device.
